# EUROPÄISCHE PATENTSCHRIFT

(11) **EP 0 331 126 B1**
(45) Veröffentlichungstag und Bekanntmachung des Hinweises auf die Patenterteilung: **04.08.1993**
(21) Anmeldenummer: 89103527.1
(22) Anmeldetag: 28.02.1989
(51) Int. Cl.: G01N 33/68, G01N 33/542, C12N 5/12, C12P 21/08

(54) **Verfahren zur Bestimmung eines Proteins nach dem Prinzip des Fluoreszenz-Polarisations Immunoassays**
Process for the determination of a protein according to the principle of fluorescense polarisation immunoassay
Procédé de détermination d'une protéine suivant le principe d'essai immunologique à polarisation de fluoresence

(30) Priorität: 29.02.1988 DE 3806430
(43) Veröffentlichungstag der Anmeldung: 06.09.1989
(73) Patentinhaber: BOEHRINGER MANNHEIM GMBH, 68298 Mannheim (DE)
(72) Erfinder: Klein, Christian, Dr. rer.nat., D-8120 Weilhelm (DE); Batz, Hans-Georg, Dr. rer.nat., D-8132 Tutzing (DE); Essig, Ulrich, Dr. rer.nat., D-8033 Planegg (DE); Naujoks, Kurt Walter, Dr. rer.nat., D-8122 Penzberg (DE)
(74) Vertreter: Huber, Bernhard, Dipl.-Chem.

(56) Entgegenhaltungen:
- EP-A- 0 210 410

## Beschreibung

Die Erfindung betrifft ein Verfahren zur Bestimmung eines Proteins nach dem Prinzip des Fluoreszenz-Polarisations-Immunoassays.

Auf dem Gebiet der Diagnostik, insbesondere im klinischen Bereich, wurden in den letzten beiden Jahrzehnten, ausgehend von Verfahren nach dem Prinzip des Radio-Immun-Assays immer neue Varianten dieser Nachweismethode entwickelt, die immer genauere und spezifischere Bestimmungen von spezifisch bindefähigen Substanzen erlauben. Als eine sehr empfindliche Nachweismethode wurde dabei der Fluoreszenz-Polarisations-Immunoassay entwickelt. Diese Methode beruht darauf, daß ein Konjugat aus einer fluoreszierenden Substanz und einem kleinen Molekül, wie beispielsweise einem Hapten, sich in Lösung sehr schnell bewegt, so daß polarisiertes Licht, das durch die Lösung geschickt wird und die fluoreszierende Substanz anregt, bei der Emission durch die zwischen Auftreffen und Emission erfolgte Drehung des Konjugats depolarisiert wird. Dabei ist der Grad der Depolarisation umgekehrt proportional der Rotationsrate des Moleküls. Bei der Bindung der fluoreszierenden Substanz an ein größeres Molekül, beispielsweise bei der Bindung des fluoreszenzmarkierten Haptens an den entsprechenden Antikörper, ist die Bewegung des gesamten Konjugates so stark eingeschränkt, daß nunmehr das polarisierte Licht von dem Zeitpunkt, wo es auf die fluoreszierte Substanz auftrifft bis zu dem Zeitpunkt wo es emittiert wird, nicht mehr depolarisiert wird, sondern polarisiert wieder aus der Lösung austritt. Die Depolarisation kann man mit an sich bekannten Vorrichtungen bestimmen. Man nutzt nun dieses Verhalten für ein Nachweisverfahren aus. Man gibt dazu in eine Lösung, in der der zu bestimmende spezifisch bindefähige Partner enthalten ist, ein Konjugat aus zu bestimmendem Partner mit fluoreszierender Substanz sowie einen Antikörper, der mit beidem, sowohl der nachzuweisenden Substanz, als auch dem fluoreszenzmarkierten Molekül bindefähig ist, zu und mißt die Abnahme der Depolarisation. Der in der Lösung vorhandene Bindungspartner und das fluoreszenzmarkierte Molekül konkurrieren dabei um den Antikörper. Die Menge an gebundener fluoreszenzmarkierter Substanz kann dann über die Abnahme der Depolarisation bestimmt werden und ist ein Maß für die Menge an zu bestimmender Substanz in der Lösung.

Wesentlich für dieses Verfahren ist die unterschiedliche Beweglichkeit des markierten Bindungspartners und des Konjugats aus markiertem Bindungspartner und Antikörper. Dies ist nur gegeben, wenn der Bindungspartner sehr klein ist. Für Haptene läßt sich das Verfahren daher sehr gut durchführen und liefert genaue Ergebnisse. Sollen jedoch größere Moleküle bestimmt werden, so reichen die Unterschiede in der Molekülgröße zwischen markiertem Molekül und Konjugat aus markiertem Molekül und Antikörper nicht mehr aus, um die Abnahme der Depolarisation ausreichend genau bestimmen zu können. Daher konnte dieses empfindliche Nachweisverfahren bisher für größere Moleküle nicht angewandt werden.

EP-A-0 210 410 offenbart ein Verfahren zur Bestimmung von größeren Molekülen (C-reaktives Protein (CRP) in Lösungen nach dem Prinzip des Fluoreszens-Polarisations-Immunoassays, in dem die Probe a) mit einem mit einer fluoreszierenden Verbindung markierten Indikator, der einen CRP - analogen Liganden mit höchstens einem gemeinsamen Epitop enthält, und b) mit einem für den Liganden und Indikator spezifischen Antikörper inkubiert wird.

Es war daher Aufgabe der Erfindung, ein Verfahren zur Verfügung zu stellen, mit dem größere Moleküle empfindlich in Lösungen nachgewiesen werden können.

Diese Aufgabe wird gelöst durch ein Verfahren zur Bestimmung eines Proteins nach dem Prinzip des Fluoreszenz-Polarisations-Immunoassays in homogenem System durch gleichzeitige Inkubation der Probelösung mit
a) einer mit einer fluoreszierenden Verbindung markierten Peptidsequenz aus 6 bis 14 Aminosäuren, von denen zwei Cystein sind, die miteinander eine Disulfidbrücke bilden, wobei die Sequenz einer Epitopsequenz des zu bestimmenden Proteins entspricht und
b) einem Antikörper, der sowohl mit dem Protein als auch mit der Peptidsequenz spezifisch bindefähig ist und für die fluoreszierende Peptidsequenz des Proteins und das native Protein molare relative Affinitäten aufweist, die sich höchstens um den Faktor 6 unterscheiden und
Messung der Depolarisation von durch die inkubierte Lösung geschicktem polarisiertem Licht.

Mit den erfindungsgemäß eingesetzten Komponenten - mit fluoreszierender Verbindung markiertes Peptid und Antikörper - gelingt es, große Moleküle in Lösung nachzuweisen. Dieser Nachweis ist sehr empfindlich. Die erfindungsgemäß verwendeten Peptidsequenzen können an ein Trägerprotein gebunden gleichzeitig auch zur Immunisierung, d.h. zur Herstellung der benötigten Antikörper, verwendet werden.

Das erfindungsgemäße Verfahren dient zur Bestimmung von Proteinen, insbesondere in Körperflüssigkeiten. Die zu bestimmenden Proteine sind immunogen wirksam und können daher auch als Antigene bezeichnet werden. Geeignet ist das Verfahren z.B. zum Nachweis von Hormonen, Enzymen und anderen biologisch aktiven hochmolekularen Substanzen. Diese Substanzen erzeugen, wenn sie einem geeigneten Organismus injiziert werden, eine Immunreaktion und führen zur Bildung von Antikörpern. Immunogen wirksam ist dabei nicht das ganze Molekül, sondern bestimmte Bereiche, die als Epitope bezeichnet werden. Dabei handelt es sich in der Regel um kleinere Bereiche, die bis zu 10 Aminosäuren umfassen und die an der Oberfläche des Moleküls liegen. Proteine weisen viele solcher Epitope an ihrer Oberfläche auf, so daß bei der Immunisierung eine Vielzahl von verschiedenen Antikörpern, die gegen die einzelnen Epitope gerichtet sind, erzeugt werden. Darüberhinaus sind auch die einzelnen Antikörper in Bezug auf ihre molare relative Affinität zu dem Antigen verschieden.

Für den Nachweis der Proteine mit hohem Molekulargewicht, die in einem Fluoreszenz-Polarisations-Immunoassay keinen analytisch auswertbaren Unterschied in der Depolarisation ergeben, werden erfindungsgemäß statt einem Konjugat aus einer fluoreszierenden Verbindung und dem Protein Konjugate eingesetzt, die aus der fluoreszierenden Verbindung und einer Peptidsequenz bestehen, die einer Epitopsequenz des zu bestimmenden Proteins entspricht. Als Epitopsequenz wird dabei eine Sequenz von 6 bis 14 Aminosäuren, von denen zwei Cystein sind, verwendet. Die beiden Cysteine bilden eine Disulfidbrücke. Falls die Epitopsequenz des zu bestimmenden Proteins mehr als zwei Cysteine aufweist, so werden bevorzugt in dem fluoreszenzmarkierten Peptid alle Cysteine bis auf zwei durch eine andere Aminosäure, bevorzugt Alanin, ersetzt, um eine definierte Disulfidbindung zu erhalten. Enthält eine Sequenz mehr als zwei Cysteine, so entsteht ein Gemisch aus Molekülen, die Disulfidbrücken an verschiedenen Stellen aufweisen. Dabei kann es zu Schwierigkeiten bei der Erkennung durch den Antikörper kommen.

Das Peptid wird in an sich bekannter Weise hergestellt. Verfahren zur Synthese von Peptiden sind allgemein bekannt und brauchen hier nicht näher erläutert zu werden.

Die Peptidsequenz wird mit einer fluoreszierenden Substanz markiert. Als fluoreszierende Verbindungen sind Verbindungen geeignet, die kurze Fluoreszenzhalbwertszeiten aufweisen, d.h. daß die Substanz nach der Anregung die Energie sehr schnell in Form von Fluoreszenz emittiert und wieder in den Ausgangszustand zurückkehrt. Geeignet sind als fluoreszierende Substanzen beispielsweise Fluoreszein und seine Derivate sowie Resorufin. Besonders bevorzugt wird Resorufin eingesetzt.

Die fluoreszierende Substanz wird in an sich bekannter Weise an die Peptidsequenz gebunden. Die Bindung kann entweder direkt zwischen den beiden Komponenten erfolgen oder aber über einen Spacer. Die Art der Bindung ist abhängig von der eingesetzten fluoreszierenden Substanz und dem verwendeten Peptid. Verfahren hierzu sind dem Fachmann bekannt.

Die Bindung der fluoreszierenden Substanz an das Peptid muß so erfolgen, daß die fluoreszierende Substanz die Epitopfunktion des Peptids nicht stört. Bevorzugt erfolgt daher die Bindung an einem der beiden Kettenenden des Peptids.

Weiterhin wird für das erfindungsgemäße Verfahren ein Antikörper eingesetzt, der sowohl mit dem zu bestimmenden Protein als auch mit dem fluoreszenzmarkierten Peptid spezifisch bindefähig ist. Um genaue Ergebnisse zu erzielen, muß er für die fluoreszenzmarkierte Peptidsequenz und für das native Protein molare relative Affinitäten aufweisen, die sich höchstens um den Faktor 6 unterscheiden. Die molaren relativen Affinitäten werden durch einen an sich bekannten Mikrotiter-ELISA bestimmt. Dazu werden mit dem nativen Protein beschichtete Mikrotiterplatten gleichzeitig jeweils mit dem Antikörper und der zu messenden Substanz (natives Protein, fluoreszenzmarkiertes Peptid) in mehreren Verdünnungsreihen inkubiert. Die zu messende Substanz inhibiert dabei die Bindung des Antikörpers an das wandgebundene Antigen je nach Affinität des Antikörpers zu der Substanz und je nach der Konzentration der zu messenden Substanz.

Die Bindung des Antikörpers wird durch Inkubation mit einem Anti-Antikörper-Enzym-Konjugat nachgewiesen, z.B. bei Spezifitätsuntersuchungen von monoklonalen Maus-Antikörpern mit einem polyklonalen Anti-Maus-Fc-Schafantikörper-Peroxidase-Konjugat. Durch Inkubation mit einem entsprechenden Enzymsubstrat wird die Bindung sichtbar gemacht.

Die zu der halbmaximalen Bindung gehörige Konzentration in Mol/Liter des zu messenden Analyten wird als molare relative Affinität bezeichnet. Je schlechter der Antikörper die zu messende Substanz bindet, um so höher ist der Wert der molaren relativen Affinität.

Die einzusetzende Antikörpermenge wird in einem Vorversuch ermittelt. Mit dem nativen Protein beschichtete Mikrotiterplatten werden analog mit dem Antikörper allein in verschiedenen Konzentrationen inkubiert und die Bindung analog gemessen. Für die oben beschriebene Messung der molaren relativen Affinität wird die Konzentration des Antikörpers eingesetzt, bei der halbmaximale Bindung beobachtet wird.

Geeignete Antikörper können beispielsweise erhalten werden, indem ein geeigneter Organismus mit dem zu bestimmenden Protein als Immunogen immunisiert wird und dann ein Antikörper nach seiner Bindungsstärke für das fluoreszenzmarkierte Peptid selektioniert wird. Ebenso kann ein derartiger Antikörper erhalten werden, indem ein geeigneter Organismus mit der Peptidsequenz als Immunogen immunisiert wird und dann der Antikörper nach seiner relativen Affinität für das native Protein selektioniert wird.

Die Selektionierung erfolgt entweder durch Immunsorption, wenn es sich um polyklonale Antikörper handelt, oder durch Screening, wenn es sich um monoklonale Antikörper handelt.

Beide Arten von Antikörpern sind für das erfindungsgemäße Verfahren geeignet. Bevorzugt werden jedoch monoklonale Antikörper eingesetzt. Da bei den erfindungsgemäßen Verfahren nur eine einzige Antikörperart eingesetzt wird, ist es wesentlich, daß der Antikörper hochspezifisch für das gewünschte Epitop ist und praktisch keine Kreuzreaktivitäten mit anderen endogenen Substanzen aufweist.

Zur erfindungsgemäßen Bestimmung eines Proteins wird eine Probelösung gleichzeitig mit dem fluoreszenzmarkierten Peptid und einem Antikörper, der für das zu bestimmende Protein und das fluoreszenzmarkierte Peptid spezifisch bindefähig ist, inkubiert. Die Änderung der Depolarisation von durch die Lösung geschicktem polarisiertem Licht wird gemessen. Diese Änderung ist ein Maß für den Anteil an gebundenem fluoreszenzmarkiertem Peptid. Aus der Menge an fluoreszenzmarkiertem Peptid, die durch Vergleich mit einer Standardkurve berechnet werden kann, wird dann die Menge an zu bestimmendem Protein in der Lösung in an sich bekannter Weise berechnet.

Ein weiterer Gegenstand der Erfindung sind die Hybridom-Zellklone 2E6 und 3C4, die am 25. Februar 1988 gemäß Budapester Vertrag bei der EUROPEAN COLLECTION OF ANIMAL CELL CULTURES (ECACC), GB, hinterlegt wurden. Sie haben die Hinterlegungsnummern erhalten: 2E6 = ECACC 88022502, 3C4 = ECACC 88022501. Aus diesen Zellinien können monoklonale Antikörper gewonnen werden, die sehr spezifisch mit dem A-Loop-Epitop des Humaninsulins sowie mit einer fluoreszenzmarkierten Sequenz dieses Epitops reagieren. Diese Antikörper eignen sich zur Verwendung für die Bestimmung von Insulin in einem Fluoreszenz-Polarisations-Bestimmungsverfahren.

Erfindungsgemäß wird ein Verfahren zur Verfügung gestellt, mit dem es möglich ist, Proteine sehr spezifisch und sehr empfindlich nachzuweisen. Weiterhin werden für das Verfahren geeignete monoklonale Antikörper zur Verfügung gestellt.

Die Erfindung wird durch die Figur und die folgenden Beispiele erläutert:

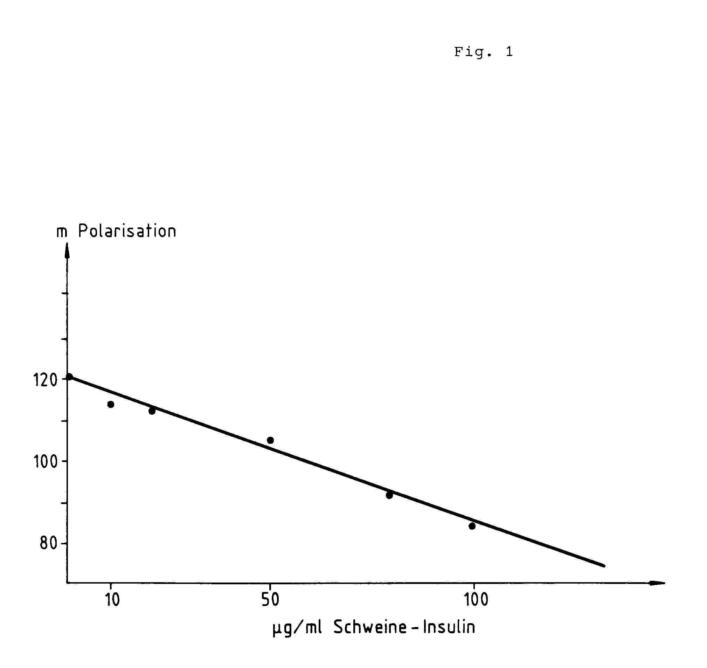

- Fig. 1: zeigt eine Eichkurve.

### Beispiel 1

Es wird eine Peptidsequenz synthetisiert, die dem h-Insulin-A-Ketten-Loop entspricht. Die Festphasensynthese wird durchgeführt in einem semiautomatischen Peptidsynthesiser der Firma Labortech, Bubendorf (Schweiz). Als N -Aminoschutzgruppe wurde die FMOC((9-Fluorenyl)-Methoxycarbonyl)-Gruppe verwendet.

Eine Beschreibung dieser Peptidsynthesemethode findet sich bei J. Meienhofer et al., Int. J. Peptide Protein Res. 13, 35-42 (1979). Die C-terminale FMOC-Aminosäure wurde, wie bei Meienhofer beschrieben, an p-Alkoxybenzylalkohol-harz (Firma Bachem, Bubendorf, Schweiz) gekoppelt.

| Syntheseprotokoll für einen Synthesezyklus: | | |
|---|---|---|
| Schritt | Zeit | Reagenz/Lösungsmittel |
| 1 | 2 x 1 min | DMF (Dimethylformamid) |
| 2 | 1 x 3 min | Piperidin/DMF 1 : 4 |
| 3 | 1 x 7 min | Piperidin/DMF 1 : 4 |
| 4 | 4 x 1/2 min | DMF |
| 5 | 2 x 1/2 min | Isopropanol |
| 6 | Stop | Ninhydrintest |
| 7 | 2 x 1 min | DMF |
| 8 | Stop | Zugabe der nächsten Fmoc-Aminosäure und HOBt (1-Hydroxybenzotriazol ) in DMF |
| 9 | 2 min | Schütteln |
| 10 | Stop | Zugabe von DCC (Dicyclohexylcarbodiimid) in DCM (Dichlormethan) |
| 11 | 90 min | Kupplung |
| 12 | 3 x 1 min | DMF |
| 13 | 2 x 1 min | Isopropanol |
| 14 | Stop | Ninhydrintest |

Als Volumen der Wasch- und Kupplungsschritte wird jeweils das 15fache des Gewichts des Startharzes verwendet. Der Ninhydrintest wird, wie bei E. Kaiser et al., Anal. Biochem. 34, 595-98 (1970) beschrieben, durchgeführt. Falls der Ninhydrintest in Schritt 14 noch freie Aminogruppen anzeigt, wird das Protokoll ab Schritt 8 wiederholt. Für die Kopplung gemäß Schritt 8 bis 11 werden die Fmoc-Aminosäure und DCC jeweils in der dreifach molaren Menge, bezogen auf die Beladung des Startharzes, eingesetzt. HOBt wird in der 4,5fachen molaren Menge eingesetzt.

Nach der Kopplung der letzten N-terminalen Fmoc-Aminosäure wird zur Abspaltung der Fmoc-Schutzgruppe Schritt 1 bis 5 des Synthesezyklus durchlaufen. Danach wird das Harz in dem 15fachen Volumen Dichlormethan (DCM)/Trifluoressigsäure (TFA)1:1 2 Stunden bei Raumtemperatur geschüttelt. Man filtriert, wäscht das Harz noch zweimal mit Dichlormethan/Trifluoressigsäure 4:1, vereinigt alle Filtrate und dampft im Vakuum unter Toluolzusatz bei 25°C ein. Der Rückstand wird mit Diethylether versetzt. Man filtriert den Feststoff ab und trocknet.

Nach dem oben angegebenen Schema wird der A-Chain-Loop 
HGluGlnCysAlaThrSerIleCysSerLeuAhxOH
S-S
synthetisiert. Als Startharz werden 5 g Fmoc Ahx = ε-Aminohexansäure-p-Alkoxybenzylalkoholharz, beladen mit 0,45 mmol/g, eingesetzt. In den Synthesezyklen werden die folgenden Fmoc-Aminosäuren nacheinander eingesetzt:

| | | |
|---|---|---|
| 1. | 2,39 g Fmoc Leu | |
| 2. | 2,59 g Fmoc Ser (tBu) | |
| 3. | 3,95 g Fmoc Cys (Trt) | |
| 4. | 2,39 g Fmoc Ile | |
| 5. | 2,59 g Fmoc Ser (tBu) | |
| 6. | 2,68 g Fmoc Thr (tBu) | |
| 7. | 2,10 g Fmoc Ala | |
| 8. | 3,95 g Fmoc Cys (Trt) | Diese Kopplungen werden jeweils einmal wiederholt. |
| 9. | 2,49 g Fmoc Gln | |
| 10. | 3,13 g Fmoc Glu (OtBu) | |
| tBu: t-Butylether Trt: Trityl OtBu: t-Butylester | | |

Vor der Abspaltung des Peptids vom Harz werden die Schutzgruppen des Cysteins abgespalten und zur Disulfidbrücke cyclisiert: Ein Gemisch aus 15 ml Hexafluorisopropanol und 45 ml DCM wird mit Jod bis zur Sättigung versetzt. Überschüssiges Jod wird über einen Faltenfilter abfiltriert. Dazu gibt man 500 mg des oben erhaltenen Peptidharzes und schüttelt 2 Stunden, filtriert und wäscht je dreimal mit DCM, DMF und Isopropanol. Danach wird, wie oben beschrieben, das Peptid vom Harz abgespalten; Ausbeute: 120 mg Rohprodukt.

Das Rohprodukt wurde an Polygosil C 18 5µ, 250x20 mm (Firma Bischoff) chromatographiert. Eluent A: Wasser mit 0,1% TFA, Eluent B: 65% Isopropanol in Wasser mit 0,1 % TFA; 20 bis 80% in 120 Minuten. Flußrate: 3,5 ml/min., Detektion bei 226 nm.

Man erhält 47 mg Reinsubstanz.
DC: (Kieselgel, Fließmittel: n-Butanol/Eisessig/Wasser 4:1:1)
Rf: 0,44
FAB-MS (fast-atom bombardment -MS) (positiv) MH⁺: 1165 (ber. 1165,5).

58 mg des erhaltenen Peptids werden in 15 ml Wasser aufgenommen und die Lösung wird mit 5%iger K₂CO₃-Lösung auf pH 8,5 eingestellt. Man gibt eine Lösung von 21,5 mg N-(Resorufinylcarbonyl)-sarcosin-N′-hydroxysuccinimidester in 15 ml Dioxan zu. Die Herstellung dieses Reagenz ist beschrieben in DE-OS 35 26 565. 20 Stunden wird bei Raumtemperatur gerührt, dann wird das Dioxan im Vakuum abgedampft und der pH mit Trifluoressigsäure auf 2,2 eingestellt. Das Konjugat aus Resorufin und Peptid fällt aus. Man saugt den Niederschlag ab und fällt dann durch Lösen in 0,1 M NaHCO₃-Lösung und Ansäuern um. Man erhält 5 mg A-Chain-Loop-Resorufin-Sarcosin. Durch Chromatographie der gesammelten Filtrate an Polygosil C18 erhält man zusätzlich 6,8 mg A-Chain-Loop-Resorufin-Sarcosin.
DC: (Kieselgel, Fließmittel, wie oben angegeben)
Rf: 0,56.

### Beispiel 2

### Gewinnung von monoklonalen Antikörpern gegen Humaninsulin

Balb/c-Mäuse, 8 bis 12 Wochen alt, werden mit 100 µg Humaninsulin an Aluminiumhydroxid und Bordetella pertussis adsorbiert, intraperitoneal immunisiert. Nach 6 Wochen werden drei weitere Immunisierungen in vierwöchigem Abstand durchgeführt. Dabei werden jeweils 50 µg Insulin an Aluminiumhydroxid und Bordetella pertussis adsorbiert, intraperitoneal verabreicht.

Ungefähr 4 Monate nach der letzten Immunisierung wird fusioniert. Vier Tage und drei Tage vor der Fusionierung wird jeweils noch einmal mit 100 µg Insulin/PBS (= phosphate buffered saline), intraperitoneal bzw. intravenös immunisiert.

Zur Fusion werden in Anlehnung an Galfré, Methods in Enzymology, 73 (1981), Seite 3, einmal 10⁸ Milzzellen einer immunisierten Maus mit 2x10⁷ Myelomzellen P3x63-Ag 8.653, ATCC-CRL 8375) gemischt und anschliessend 10 Minuten zentrifugiert (300 g, 4°C). Die Zellen werden noch einmal mit BSS (= balanced salt solution) gewaschen und bei 400 g zentrifugiert. Der Überstand wird entfernt. Das Zellsediment wird mit 1 ml 60%iger PEG-Lösung (MG 4000, Merck) versetzt. Danach wird bei Raumtemperatur 5 ml RPMI 1640-Medium (RPM = Rosewell Parker Memory Institut) ohne fötales Kälberserum (FKS), anschließend noch einmal 5 ml RPMI 1640-Medium, mit 10% FKS langsam zugetropft, mit Medium auf 50 ml aufgefüllt und 10 Minuten bei 400 g zentrifugiert. Die sedimentierten Zellen werden in RPMI 1640-Medium mit 10% FKS aufgenommen. Je 2x10⁵ Milzzellen werden auf 24-well-Zellkulturplatten (Firma Nunc) eingesät. Zu jeder Kultur werden 1x10⁵ Milzzellen oder 5x10⁴ Peritoneal-Exsudat-Zellen als Futterzellen zugefügt. Am folgenden Tag wird Hypoxanthin-Azaserin-Selektionsmedium (100 mM Hypoxanthin, 1 µg/ml Azaserin) zugegeben.

Nach ca. 7 bis 10 Tagen werden bereits viele Klone sichtbar. Der Überstand der Primärkulturen wird nach einem in Beispiel 3 beschriebenen ELISA-Verfahren getestet. Primärkulturen, die Antigen-spezifische Antikörper enthalten, werden mit Hilfe eines fluoreszenzaktivierenden Zellsorters auf 96-well-Zellkulturplatten (Firma Nunc) weiter kloniert. Als Futterzellen dienen 1x10⁴ Peritoneal-Exsudat-Zellen oder 2x10⁴ Milzzellen pro 96er-well der Kultur.

Auf diese Weise konnten beispielsweise die Hybridom-Zellklone 2E6 (ECACC 88022502) und 3C4 (ECACC 88022501) isoliert werden.

Zur Erzeugung von Ascites werden 5x10⁶ Hybrid-Zellen intraperitoneal Mäusen gespritzt, die zuvor ein- bis zweimal mit 0,5 ml Pristan vorbehandelt worden sind. Eine bis drei Wochen danach kann aus den Mäusen Ascites-Flüssigkeit gewonnen werden. Hieraus können in üblicher Weise die Antikörper isoliert werden. Diese monoklonalen Antikörper sind spezifisch gegen Insulin gerichtet und zeigen keine bzw. nur eine geringe Kreuzreaktivität mit Pro-Insulin.

### Beispiel 3

### Screening-Test auf Antikörper gegen Insulin

Um die Anwesenheit und Spezifität von Antikörpern gegen Insulin im Serum immunisierter Mäuse oder in dem Kulturüberstand der Hybridzellen oder in Ascites zu erkennen, wird ein ELISA-Verfahren als Testprinzip verwendet: Mikrotiterplatten werden mit 1 µg Insulin/ml Beschichtungspuffer (0,2 M Natriumcarbonat/-Bicarbonat, pH 9,3 bis 9,5) bei 37°C über Nacht beschichtet. Es wird 10 Minuten mit 0,9% Natriumchloridlösung und 1% Albuminlösung nachbehandelt. Danach wird mit 0,9% Natriumchloridlösung gewaschen. Anschließend wird bei 37°C eine Stunde mit 100 µl Probe inkubiert und erneut mit 0,9% Natriumchloridlösung gewaschen. Es folgt eine weitere Inkubation (eine Stunde bei 37°C) mit 100 bis 150 mU/ml eines Schaf-anti-Maus-IgG-Peroxidase-Konjugats. Nach einem erneuten Waschschritt mit 0,9% Natriumchlorid wird die Peroxidaseaktivität in üblicher Weise bestimmt (beispielsweise mit ABTS, 30 Minuten bei Raumtemperatur, abgelesen wird die Extinktionsdifferenz, ΔmE bei 405 nm).

Der ELISA-Test kann auch wie folgt durchgeführt werden:
Die Mikrotiterplatten werden zunächst mit einem Schafanti-Maus-IgG beschichtet (20 bis 30 µg/ml Beschichtungspuffer, eine Stunde bis über Nacht, 37°C). Danach wird wie oben beschrieben, weiter behandelt, die Probelösung zugesetzt und erneut gewaschen. Schließlich wird mit 250 mU/ml eines Insulin-Peroxidase-Konjugat inkubiert (1 Stunde, 37°C). Es wird erneut gewaschen und die Peroxidase-Aktivität, beispielsweise mit ABTS bestimmt.

Die Antikörper werden zusätzlich auf die Erkennung des Insulin-Peptids wie im Beispiel 4 beschrieben, gescreent.

### Beispiel 4

### Materialien

- Mikrotiterplatten:: A: NUNC 4-42404 II
B: NUNC 2-69620
- 12-Kanal-Pipette:: Dynatech, Katalog-Nr. 77-887-00
- Plattenschüttler:: Flow Laboratories, Titertek, Katalog-Nr. 77-471-00
- Abdeckfolien:: Dynatech Plate Sealers, Katalog-Nr. M 30
- ELISA-Reader:: Dynatech MR 700
- Beschichtungspuffer:: 50 mM Natriumcarbonat, pH 9,6
- Probenpuffer:: 10 mM Natriumphosphat, pH 7,4, 0,9% NaCl, 0,1% Tween 20, 1% Crotein C
- Waschpuffer:: 0,9% NaCl, 0,1% Tween 20
- Antikörper-Enzym-Konjugat:: Konjugat aus Peroxidase und dem Fab-Fragment eines polyklonalen Antikörpers aus Schaf, der gegen den Fcγ-Teil von Maus-IgG gerichtet ist, 113 mU/ml in Probenpuffer
- Substrat:: 100 mmol/l Phosphat-Citrat-Puffer pH 4,4
3,2 mmol/l Natriumperborat
1,9 mmol/l ABTS (2,2′-Azino-di-/3ethylbenzthiazolin-sulfonsäure(6)/diammoniumsalz)
- Antikörper:: MAK gegen Insulin 2E6 bzw. 3C4
- Peptidderivat:: A-Chain-loop (Herstellung nach Bsp. 1) A-Chain-loop-Resorufin-Sarcosin (Herstellung nach Bsp. 1)
- Antigen:: Human-Insulin

In einem Vorversuch werden die in die eigentlichen Spezifitätsexperimente einzusetzenden Antikörpermengen bestimmt.

Hierzu werden Mikrotiterplatten mit Insulin beschichtet. Je 100 µl Napf werden 2,5 µg Insulin pro ml Beschichtungspuffer eine Stunde lang bei Raumtemperatur inkubiert. Danach wird die Lösung abgesaugt und dreimal mit Waschpuffer gewaschen.

Anschließend werden dem festphasengebundenen Insulin Verdünnungsreihen der Antikörper 2E6 bzw. 3C4 (Ascites) zugegeben, wobei die Verdünnung mit Probenpuffer ab 1:100 in Fünferschritten erfolgt. Es jeweils eine Stunde bei Raumtemperatur inkubiert und anschliessend gewaschen.

Die an Insulin gebundenen Antikörper werden durch Zugabe eines Konjugats aus Peroxidase und dem Fab-Fragment eines polyklonalen Antikörpers aus Schaf, der gegen den FCγ-Teil von Maus-IgG gerichtet ist, über die Reaktion der Peroxidase mit zugegebenem Substrat bestimmt. Die Nachweisreaktion wird durch Zugabe des Substrats in alle Näpfe gestartet. Die Messung erfolgt im ELISA-Reader bei 405 nm (Referenzwellenlänge 490 nm).

Als Titer wird diejenige Antikörperverdünnung definiert, bei der eine halbmaximale Bindung stattfindet. Diese Antikörpermenge wird in die folgenden Versuche eingesetzt.

Die Spezifität der monoklonalen Antikörper wird untersucht. Dazu werden die Reaktivitäten einzelner Antikörper mit verschiedenen in Lösung befindlichen Komponenten verglichen.

In mit 1% Crotein C vorbeschichtete Mikrotiterplatten werden je 50 µl einer Lösung des monoklonalen Antikörpers in doppelter Titerkonzentration und 50 µl Antigen-Lösung bzw. Lösung des Peptidderivats (Verdünnungsreihe, siehe unten) pipettiert und gemischt und 30 Minuten bei Raumtemperatur inkubiert. Danach werden 100 µl Aliquots der Gemische in die mit Insulin beschichteten Mikrotiterplatten transferiert.

### Verdünnungsreihen:

| Human-Insulin | |
|---|---|
| A-Chain-loop | ab 10 µg/ml Probenpuffer mit Probenpuffer in 5er-Schritten |
| A-Chain-loop-Resor.-Sark. | |

Die zur halbmaximalen Bindung gehörige Konzentration in mol/l einer zu bestimmenden Substanz wird als molare relative Affinität definiert.

Die molare relative Affinität der MAKs 2E6 bzw. 3C4 gegenüber dem unmarkierten Peptid aus Beispiel 1 ist um den Faktor 5 höher als gegenüber Humaninsulin, die molare relative Affinität gegenüber dem resorufinmarkierten Peptid aus Beispiel 1 ist um den Faktor 1,8 höher als gegenüber Humaninsulin. Daraus folgt, daß jeweils das Peptidderivat etwas schwächer als Humaninsulin erkannt wird.

### Beispiel 5

### Insulin-Bestimmung mittels FPIA-Messung

1935 µl 0,1 M Kaliumphosphatpuffer pH 7,8 werden mit 20 µl Probe, 20 µl Ascites-Lösung des Klons 2E6 (6,4 · 10⁻⁶ mol/l IgG) sowie 25 µl einer Lösung des resorufinmarkierten Peptides aus Beispiel 1 versetzt.

Nach fünfminütiger Inkubation bei 37°C wird die Fluoreszenzpolarisation gemessen (Anregungswellenlänge: 575 nm, Emissionswellenlänge 594 nm.

Fluoreszenzspektrometer F 4.000, Hitachi, mit Polarisationsmeßkopf.

Die so erhaltene Eichkurve ist in Fig. 1 gezeigt. Jeder Meßpunkt entspricht dem Mittelwert aus drei Einzelmessungen.

## Patentansprüche

1. Verfahren zur Bestimmung eines Proteins nach dem Prinzip des Fluoreszenz-Polarisations-Immunoassays in homogenem System durch gleichzeitige Inkubation der Probelösung mit
a) einer mit einer fluoreszierenden Verbindung markierten Peptidsequenz aus 6 bis 14 Aminosäuren, von denen zwei Cystein sind, die miteinander eine Disulfidbrücke bilden, wobei die Sequenz einer Epitopsequenz des zu bestimmenden Proteins entspricht und
b) einem Antikörper, der sowohl mit dem Protein als auch mit der Peptidsequenz spezifisch bindefähig ist und für die fluoreszierende Peptidsequenz des Proteins und das native Protein molare relative Affinitäten aufweist, die sich höchstens um den Faktor 6 unterscheiden und
Messung der Depolarisation von durch die inkubierte Lösung geschicktem polarisiertem Licht.

2. Verfahren nach Anspruch 1, **dadurch gekennzeichnet,** daß man ein Peptid verwendet, welches endständig die Fluoreszenzmarkierung trägt.

3. Verfahren nach einem der Ansprüche 1 oder 2, **dadurch gekennzeichnet,** daß man einen Antikörper verwendet, der durch Immunisierung mit dem nativen Protein als Immunogen hergestellt und nach seiner molaren relativen Affinität zu der Peptidsequenz selektioniert wurde.

4. Verfahren nach einem der Ansprüche 1 oder 2, **dadurch gekennzeichnet,** daß man einen Antikörper verwendet, der durch Immunisierung mit der Peptidsequenz als Immunogen hergestellt und nach seiner molaren relativen Affinität für das native Protein selektioniert wurde.

5. Verfahren nach einem der vorhergehenden Ansprüche, **dadurch gekennzeichnet,** daß man einen monoklonalen Antikörper verwendet.

6. Hybridom-Zellklon 3C4 mit der Hinterlegungsnummer ECACC 88022501.

7. Hybridom-Zellklon 2E6 mit der Hinterlegungsnummer ECACC 88022502.

## Claims

1. Process for the determination of a protein according to the fluorescence polarisation immunoassay principle in a homogeneous system by simultaneous incubation of the sample solution with
a) a peptide sequence, labelled with a fluorescing compound, of 6 to 14 amino acids, two of which are cysteine which form a disulphide bridge with one another, the sequence thereby corresponding to an epitope sequence of the protein to be determined, and
b) an antibody which is specifically bindable not only with the protein but also with the peptide sequence and displays for the fluorescing peptide sequence of the protein and the native protein molar relative affinities which differ by a factor of at most 6, and
measurement of the depolarisation of polarised light passed through the incubated solution.

2. Process according to claim 1, wherein a peptide is used which carries the fluorescence labelling terminally.

3. Process according to claim 1 or 2, wherein an antibody is used which has been produced by immunisation with the native protein as immunogen and has been selectioned according to its molar relative affinity to the peptide sequence.

4. Process according to claim 1 or 2, wherein an antibody is used which has been produced by immunisation with the peptide sequence as immunogen and has been selectioned according to its molar relative affinity for the native protein.

5. Process according to any of the preceding claims, wherein a monoclonal antibody is used.

6. Hybridoma cell clone 3C4 with the accession number ECACC 88022501.

7. Hybridoma cell clone 2E6 with the accession number ECACC 88022502.

## Revendications

1. Procédé de détermination d'une protéine selon le principe de l'essai immunologique à polarisation de fluorescence dans un système homogène par incubation simultanée de la solution échantillon avec
a) une séquence peptidique marquée avec un composé fluorescent et constituée par 6 à 14 acides aminés parmi lesquels deux sont des cystéines qui forment entre elles un pont disulfure, la séquence correspondant à une séquence d'épitope de la protéine qui doit être déterminée, et
b) un anticorps qui est capable de se lier de manière spécifique tant avec la protéine qu'avec la séquence peptidique et qui présente pour la séquence peptidique fluorescente de la protéine et pour la protéine native des affinités molaires relatives qui diffèrent entre elles au plus d'un facteur 6 et
par mesure de la dépolarisation de la lumière polarisée envoyée dans la solution incubée.

2. Procédé selon la revendication 1, caractérisé en ce que l'on utilise un peptide qui porte le marquage par fluorescence à une extrémité.

3. Procédé selon l'une des revendications 1 ou 2, caractérisé en ce que l'on utilise un anticorps qui a été préparé par immunisation avec la protéine native en tant qu'immunogène et qui a été sélectionné d'après son affinité molaire relative vis à vis de la séquence peptidique.

4. Procédé selon l'une des revendications 1 ou 2, caractérisé en ce que l'on utilise un anticorps qui a été préparé par immunisation avec la séquence peptidique en tant qu'immunogène et qui a été sélectionné d'après son affinité molaire relative vis à vis de la protéine native.

5. Procédé selon l'une des revendications précédentes, caractérisé en ce que l'on utilise un anticorps monoclonal.

6. Clone de cellules d'hybridome 3C4 de numéro de dépôt ECACC 88022501.

7. Clone de cellules d'hybridome 2E6 de numéro de dépôt ECACC 88022502.
